# EUROPEAN PATENT APPLICATION

(11) **EP 4 447 067 A1**
(43) Date of publication of application: **16.10.2024**
(21) Application number: 23167837.6
(22) Date of filing: 13.04.2023
(51) Int. Cl.: G16H 50/30, G16H 50/20, A61B 5/00, A61C 17/00, A61C 17/22, G06N 20/00, A46B 15/00

(54) **MACHINE LEARNING TECHNIQUES FOR PREDICTIVE PERIODONTITIS ASSESSMENT USING MEASUREMENT DATA CAPTURED BY A PERSONAL ORAL HEALTH CARE DEVICE**

(71) Applicant: Koninklijke Philips N.V., 5656 AG Eindhoven (NL)
(72) Inventor: KOOIJMAN, Gerben, Eindhoven (NL); CIUHU, Calina, Eindhoven (NL); SCHEFFERS, Lucas Petrus Henricus, Eindhoven (NL); MASCULO, Felipe Maia, Eindhoven (NL); OP DEN BUIJS, Jorn, Eindhoven (NL)
(74) Representative: Philips Intellectual Property & Standards

(57) **Abstract**

The invention provides a machine learning model structure for predictive periodontitis assessment using a divide-and-conquer technique, comprising a first machine-learning model and a second machine-learning model, wherein the first machine-learning model is adapted to predict a health status of a user regarding formation of gingivitis based at least in part on measurement data captured by a personal oral health care device used by the user and wherein the second machine-learning-model is adapted to predict a health status of the user regarding formation of periodontitis based at least in part on a predicted health status of the user regarding formation of gingivitis obtained using the first machine-learning model.

## Description

### FIELD OF THE INVENTION

The present invention generally concerns the field of personal oral health care devices, in particular smart electronic oral hygiene devices such as toothbrushes, or interdental cleaners, and the like. The invention generally relates to predictive assessment of oral health development of a user as well as to preventive measures avoiding unsatisfactory and detrimental oral hygiene and severe medical conditions such as periodontitis by employing a smart personal oral health device.

### BACKGROUND OF THE INVENTION

Digitalization is ubiquitous in today's world and fundamentally changes the way we live and communicate. One application domain of digitalization concerns personal care or health devices. For example, a user may use a smart toothbrush, a smart interdental cleaner, or the like. Such devices may be equipped with means to provide an improved brushing or cleaning behavior. Furthermore, certain indications and/or instructions could be provided to the user, i.e., in order to guarantee a good oral cleaning behavior and hygiene. For example, a toothbrush may provide indications regarding specific recommended brushing durations. This can help ensuring that a user brushes his teeth, or certain mouth regions, for a too short or even for a too long time.

Yet, oral diseases still pose great problems to many users of modem oral health care devices. Amongst others, periodontitis is a serious form of periodontal disease that may result in severe health consequences, both on an oral as well as systemic level (e.g., by way of comorbidities).

The problem related to periodontitis is hence much bigger, going way beyond just being unpleasant. Furthermore, on an economic level, the occurrence of diseases that necessitates professional treatment poses a burden on the patients, the health care professionals and providers, but also the economy as a whole as well as specifically on insurers.

Periodontitis (sometimes abbreviated as Perio) may develop gradually, chronically and progressively. Therefore, due to its nature to manifest itself insidiously as well as unnotedly, high caution is required. It is therefore difficult to predict, and even more difficult to prevent.

Already proper diagnose may pose problems: One key reason is that the default screening process involves partial or full mouth pocket probing, which is a time consuming and potentially painful process that can only be done by a dental professional.

Gingivitis is a usually regarded a non-destructive and rather superficial disease that causes inflammation of the gums. It is usually reversible. Gingivitis is hence rather mild in nature. It may later result in periodontitis, but does not have to. Due to this mild nature, the mere occurrence of gingivitis is usually not regarded as a warning sign for the more severe periodontitis by the population as well as the dental health care professionals. The fact that periodontitis is always preceded by gingivitis is hence poorly exploited for preventive measures.

Furthermore, though being a mild disease, also forms of gingivitis should and can be - as is known - avoided by a good and/or adapted oral cleaning behavior. A goal of responsible oral health care should be a form of responsible health care in the short as well as the intermediate and long term. It is hence not desirable to e.g., tolerate repeated occurrences and suffering from gingivitis. The known solutions clearly fall short to unfold their advantages to the user health on all different time scales simultaneously.

Tremendous efforts have been made to provide a fully ab initio data-driven model for prediction of periodontitis. Although tremendous amounts of data are available today, their computational combination to meaningful insights that can be computed in finite time and with finite resources and readily be exploited remains an ongoing challenge.

Big amounts of data and the potential hiding therein remain therefore unused, limiting the potential of meaningful analysis as well as consecutive action.

Such data comprises, for example, the National Health And Nutrition Examination Survey (NHANES) conducted in the United States by the Center for Disease Control and Prevention (CDC).

The known techniques for analysis and prevention of oral diseases therefore needs to be improved.

### SUMMARY OF THE INVENTION

A solution to the problem has now been devised by the subject-matter of the independent claims. Accordingly, a computer-implemented method for predictive periodontitis assessment is provided as defined in independent claim 1. The method may involve and/or be based on measurement data captured by a personal oral health care device used by a user. The method may involve predicting a health status of a user regarding formation of gingivitis based at least in part on measurement data captured by a personal oral health care device used by the user. This may be done using a first machine-learning model. The method may involve predicting a health status of the user regarding formation of periodontitis based at least in part on the predicted health status of the user regarding formation of gingivitis. This may be done using a second machine-learning-model.

The division and/or distribution of the task to the first machine-learning-model and the second machine-learning-model may be seen as a divide-and-conquer strategy that renders the solution to the task feasible. A machine learning model structure for predictive periodontitis assessment may comprise a first machine-learning model and a second machine-learning model. The first machine-learning model may be adapted to predict a health status of a user regarding formation of gingivitis based at least in part on measurement data captured by a personal oral health care device used by the user. The second machine-learning-model may be adapted to predict a health status of the user regarding formation of periodontitis based at least in part on a predicted health status of the user regarding formation of gingivitis obtained using the first machine-learning model.

A method of the present invention may be carried out by and/or on a personal oral health care device, particularly a toothbrush, a data processing device for a personal oral health care device, a controller for a data processing device for a personal oral health care device, a computer and/or distributed computer networks such as computing clouds. An embodiment of the present invention may also be a computer program product.

Due to the two-part model structure employing a first machine-learning model as well as a second machine-learning model, the difficult task becomes manageable at all. This approach may be seen as a divide-and-conquer strategy. The first machine-learning model may predict a user's health status as to gingivitis in a reliable manner based on the measurement data taken by a personal oral health care device, such as a toothbrush. The second machine-learning model may then use this result. It hence draws on this previous finding to reliably produce a user's predicted health status with respect to formation of periodontitis. The second machine-learning model may employ big data sets, such as the National Health And Nutrition Examination Survey (NHANES) conducted in the United States by the Center for Disease Control and Prevention (CDC). Other factors than oral hygiene may play important roles. For example, such data may be selected based on the presence of a proven or suspected link with periodontal disease/oral health (e.g., smoking habit), or a relation with inflammation, or a direct relation with oral health (dental examination data).

With the present invention, due to enhanced predictive knowledge, preventive actions can be taken. These may comprise preventive actions by the user as well as the health care practitioner or provider. These may also comprise automatic adaptations of the health care delivered, e.g., automatic adaptations in the cleaning or brushing behaviour of a user's toothbrush or his or her interdental cleaner.

The real brushing behavior of a user, in particular objectively measured brushing behavior and/or brushing behavior from a technical viewpoint, may be used as a starting point for the predictive analysis. This results in a high accuracy and precision. The first machine-learning model may hence be conceived as relying on rather short-term data. By using real measurements as inputs as part of a direct or indirect measurement method, the method according to the above-described aspects predicts the physical state of an existing real object, namely the health status of the user regarding formation of gingivitis, and eventually regarding formation of periodontitis.

The second machine-learning model may be a model trained by supervised learning. This model may involve a wide pre-selection of features which are later condensed, for example using extensive feature correlation analysis. The model may use as additional input non-longitudinal data and/or longitudinal data. Non-longitudinal data is more readily available. However, longitudinal data may lead to improved results. The model may involve generation of quasi-longitudinal data from non-longitudinal data. The proposed dataset modification and/or enhancement could, for example, be done on the basis of age groups.

The present invention also provides a computer program, or a computer-readable medium storing a computer program. The computer program may comprise instructions which, when the program is executed on a computer and/or a computer network, cause the computer and/or the computer network to carry out any of the methods disclosed herein.

The present invention also provides a machine-learning model data structure. The data structure may embody a machine-learning model, in particular a machine-learning model, for compressing/encoding and/or decoding. The model may be trained, or additionally prepared, using any of the methods disclosed herein.

Measurement data may be captured by an oral health care device. This may comprise personal brushing data of a user's toothbrush, his or her interdental cleaner, and the like. The data may include data related to one or more brushing session(s) performed by the user. Such measurement data may comprise, by way of non-limiting examples, measured brushing frequency, brushing duration, brushing angle, brushing pressure, scrubbing behavior, and coverage of tooth segments. In some embodiments, these parameters can be dynamically adapted to allow for health status improvement, based on the models' results. Plaque measurements offer a suitable extension of the measurement data, due to the high correlation with respect to gingivitis (first machine-learning model). By restricting the direct use of the measurement data to the first machine-learning model (i.e., excluding the second machine-learning model), the computational complexity of the model may be significantly reduced, without detrimental effect of the obtained results. Another extension may be provided by direct gingivitis measurements. Additional user information may play a role. This may comprise data about demographics and/or comorbidities.

The results may be used for changing and/or outputting control signals for adapting e.g., the behavior of a personal oral health care device and/or outputting data or indications to a user, such as causing of an alarm or outputting of a recommendation. Parameters such as duration or intensity of brushing, speed, pressure and/or head orientation may be adapted accordingly.

In a preferred embodiment, the operation and training of the first machine-learning model are performed in real-time. This could happen in real-time already during operation of the personal health device. Alternatively, it could happen shortly afterwards, e.g. in between sessions, e.g. before the next session of operation. In this way, the first machine-learning model will flexibly and successfully model the accurate short-timescale behaviour needed for the predictive health status assessment as to gingivitis.

The first machine-learning model may be small, handy and kept flexible, modeling short-timescale behaviour and being appropriately reactive to users' behavior changes, whereas the second machine-learning model may be trained extensively, e.g., in a computing cloud with high availability of computational power, to achieve best results.

Aspects of the invention may be employed by insurance companies as well as health care providers. It may be used to reduce cost as well as financial risk, and to render cost estimates and predictions more reliable due to the enhanced medical predictive insight related to health status quo as well as a likely future health status. Predictive statistics can be leveraged as well to a new and unprecedented level.

### BRIEF DESCRIPTION OF THE DRAWINGS

The disclosure may be better understood by reference to the following drawings:
Fig. 1: A flow diagram illustrating several steps in the development of periodontitis as well as structural aspects of the machine-learning model,
Fig. 2: A flow diagram illustrating additional aspects of a possible embodiment of the present invention,
Fig. 3: A plot showing a receiver operating characteristic (true positive - false positive) curve resulting from an implementation of predictive periodontitis assessment,
Fig. 4: A plot showing a graph which indicates how well the model is calibrated by comparing the true prevalence of disease with the model-predicted risk of disease using an implementation of the inventive model.

### DETAILED DESCRIPTION OF THE EMBODIMENTS

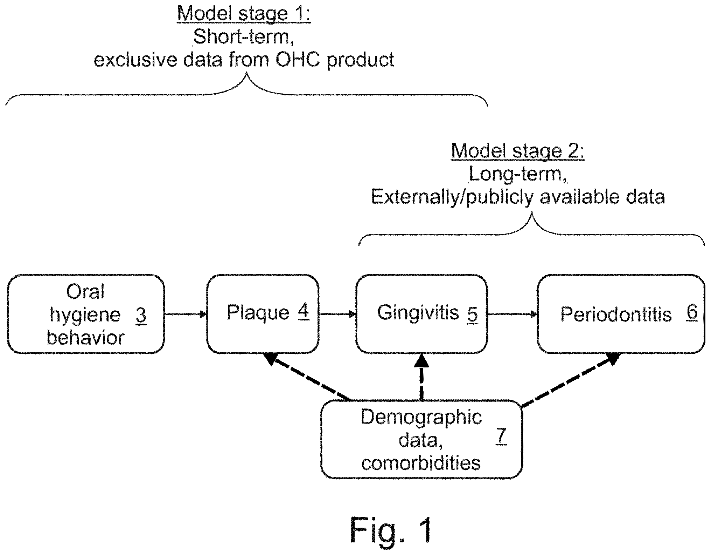

Fig. 1 shows a flow diagram illustrating several steps in the development of periodontitis as well as structural aspects of the machine-learning model. A first model stage 1 may correspond to modelling accomplished by a first machine-learning model. A second model stage 2 may correspond to modelling accomplished by a second machine-learning model. As can be seen, a common pattern in the progressive development of periodontitis is that a certain oral hygiene behavior 3 of a user (e.g., us of a toothbrush, an interdental cleaner, air floss) may lead to plaque 4 that may provoke, together with other factors, in a resulting development of gingivitis 5. Gingivitis 5 may lead to periodontitis 6.

Demographic data 7 as well as data on comorbidities 7 may play an important role in modelling of the steps 4, 5, 6. The first model stage 1 may be trained on-the-fly and/or in real-time, based on measurement data taken that relates to the brushing and cleaning behavior and/or oral hygiene of a user. Development of plaque 4 may be modelled by the model or, additionally, direct measurement data on plaque may be available and used.

A predicted health status (such as a risk) for gingivitis will be used against a second model stage 2 that may, based on long-term and statistical data, e.g., from NHANES databases, to compute a predicted health status for perio.

A feature engineering may be performed that optimizes the number of relevant features for a practical calculation. For example, available features may be clustered in clusters of highly correlated features, with relatively lower correlations between the clusters.

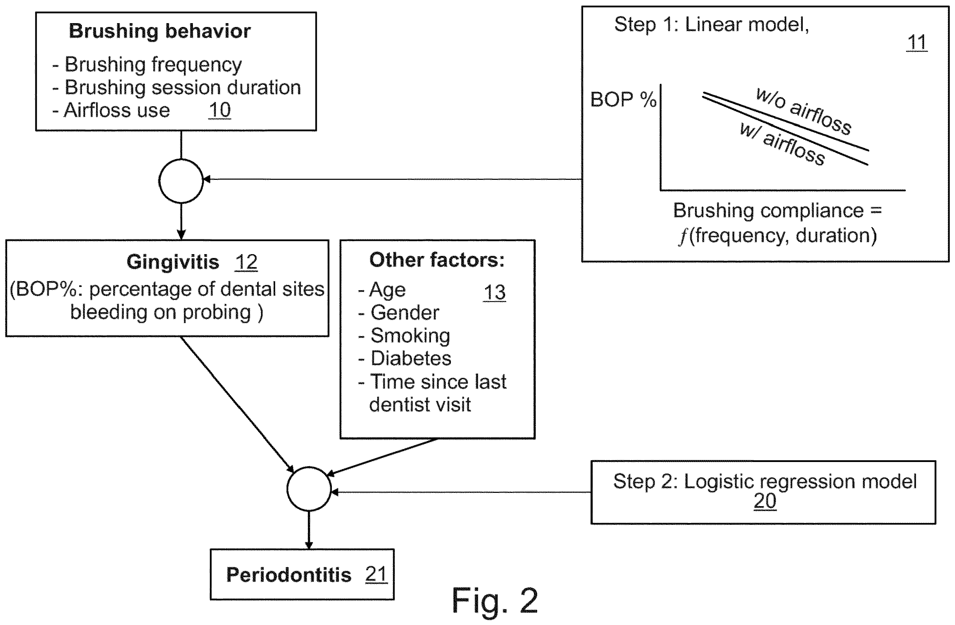

Fig. 2 shows flow diagram illustrating additional aspects of a possible embodiment of the present invention.

In implementing a first model stage 1, a linear model 11 may be used. Different regression curves may be based on presence or absence of airfloss usage. Indicator quantities for gingivitis 12 such as occurrence of Bleeding On Probing (BOP%) may be computed. BOP% may relate to a percentage of dental sites that bleed upon probing. User measurement data 10 such as, for example, brushing frequency (e.g., how often a specific user brushes his teeth per day), brushing session duration (e.g., duration in seconds) and/or airfloss use may be used as model input.

At the second model stage 2, additional factors 13 may be evaluated and/or used as model input, such as age (e.g., in years), gender, smoking (yes/no), diabetes and/or a time that has lapsed since the last visit at the oral healthcare professional (e.g., a dentist). A logistic regression model 20 may be employed to provide a predictive health status regarding perio 21.

This may be used for changing one or more settings on the oral health care device, e.g., the toothbrush or the interdental cleaner, to avoid development of perio and improve the oral hygiene.

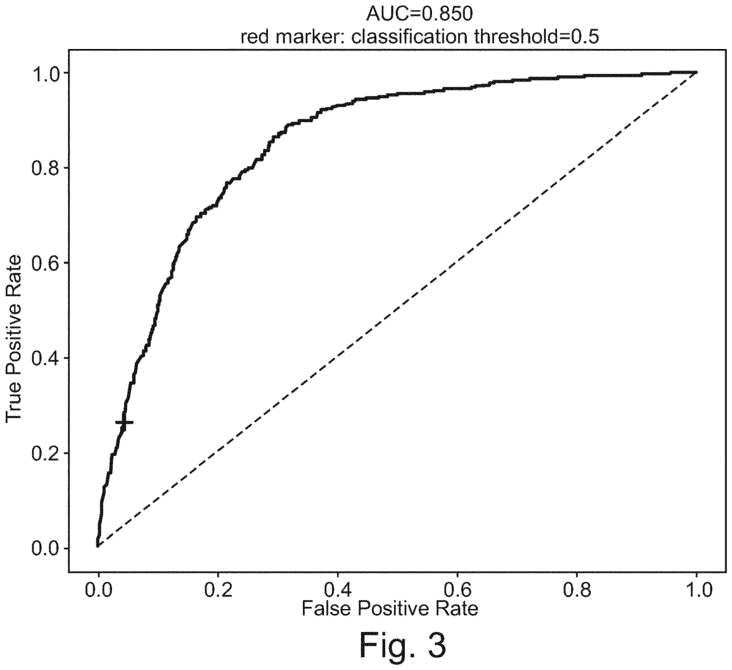

Fig. 3 shows a plot showing a receiver operating characteristic (true positive - false positive) curve resulting from an implementation of predictive periodontitis assessment. The True Positive Rate is plotted against the False Positive Rate, demonstrating the success and quality achieved by an embodiment of the inventive model.

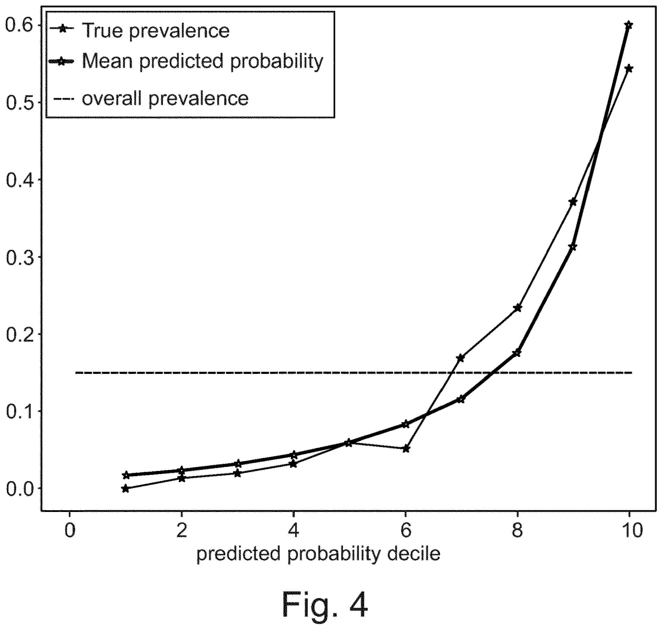

Fig. 4 shows a plot showing a graph which indicates how well the model is calibrated by comparing the true prevalence of disease with the model-predicted risk of disease using an exemplary implementation of an embodiment of the inventive model. The curves show good agreement between true prevalence as well as averaged predicted probability, over all 10 deciles shown on the abscissa. The dashed line indicates an overall prevalence.

On a more global level, the model may also be used for insightful statistical purposes. For example, without recourse to large amounts of data, one may e.g., determine the reduction of risk (e.g., x %) that is achieved e.g., by brushing the teeth twice, or three times, per day (brushing frequency).

The embodiment(s) discussed in the figure descriptions may be combined with any of the preferred additional features for the invention disclosed herein.

Although some aspects have been described in the context of a device, it is clear that these aspects also represent a description of the corresponding process, where a block or device corresponds to a process step or a function of a process step. Similarly, aspects described in the context of a process step also constitute a description of a corresponding block or element or feature of a corresponding device. Also, features disclosed in the context of a certain process, device or model may be used in a corresponding other process, device or model. For example, features of the encoder model may be used analogously in a corresponding decoder model, and vice versa.

Embodiments of the invention may be implemented in a computer system. The computer system may be a local computing device (e.g., personal computer, laptop, tablet computer, or cell phone) having one or more processors and one or more storage devices, or may be a distributed computing system (e.g., a cloud computing system having one or more processors or one or more storage devices distributed at various locations, for example, at a local client and/or one or more remote server farms and/or data centers). The computer system may comprise any circuitry or combination of circuitry. In one embodiment, the computer system may comprise one or more processors, which may be of any type. As used herein, processor may mean any type of computing circuit, such as, but not limited to, a microprocessor, a microcontroller, a complex instruction set microprocessor (CISC), a reduced instruction set microprocessor (RISC), a very long instruction word (VLIW; VLIW) microprocessor, a graphics processor, a digital signal processor (DSP), a multi-core processor, a field-programmable gate array (FPGA), or any other type of processor or processing circuit. Other types of circuitry that may be included in the computer system may include a custom-built circuit, an application-specific integrated circuit (ASIC), or the like, such as one or more circuits (e.g., a communications circuit) for use in wireless devices such as cellular phones, tablet computers, laptop computers, two-way radios, and similar electronic systems. The computer system may include one or more storage devices, which may include one or more storage elements suitable for the particular application, such as main memory in the form of random access memory (RAM), one or more hard drives, and/or one or more drives that handle removable media, such as CDs, flash memory cards, DVDs, and the like. The computer system may also include a display device, one or more speakers, and a keyboard and/or controller, which may include a mouse, trackball, touch screen, voice recognition device, or any other device that allows a system user to input information to and receive information from the computer system.

Some or all of the method steps may be performed by (or using) a hardware device, such as may be a processor, a microprocessor, a programmable computer, or an electronic circuit. In some embodiments, one or more of the key method steps may be performed by such a device.

Depending on certain implementation requirements, embodiments of the invention may be implemented in hardware or software. The implementation may be performed using a non-volatile storage medium such as a digital storage medium, such as a floppy disk, a DVD, a Blu-Ray, a CD, a ROM, a PROM and EPROM, an EEPROM, or a FLASH memory, on which electronically readable control signals are stored that interact (or are capable of interacting) with a programmable computer system such that the respective process is performed. Therefore, the digital storage medium may be computer readable.

Some embodiments according to the invention include a data carrier having electronically readable control signals that can interact with a programmable computer system so as to perform any of the methods described herein.

In general, embodiments of the present invention may be implemented as a computer program product having program code, the program code being effective to perform any of the methods when the computer program product is running on a computer. For example, the program code may be stored on a machine-readable medium.

Further embodiments include the computer program for performing any of the methods described herein stored on a machine-readable medium.

In other words, an example embodiment of the present invention therefore includes a computer program having program code for performing any of the methods described herein when the computer program is running on a computer.

Thus, another embodiment of the present invention is a storage medium (or a digital storage medium or a computer-readable medium) comprising a computer program stored thereon for performing any of the methods described herein when executed by a processor. The data carrier, digital storage medium, or recorded medium is generally tangible and/or non-transitory. Another embodiment of the present invention is an apparatus as described herein comprising a processor and the storage medium.

Thus, another embodiment of the invention is a data stream or signal sequence representing the computer program for performing any of the methods described herein. For example, the data stream or signal sequence may be configured to be transmitted over a data communication link, such as over the Internet.

Another example embodiment comprises a processing means, for example, a computer or programmable logic device, configured or adapted to perform any of the methods described herein.

A further example embodiment comprises a computer having installed thereon the computer program for performing any of the methods described herein.

Another embodiment according to the invention comprises a device or system configured to transmit (for example, electronically or optically) a computer program for performing any of the methods described herein to a receiver. The receiver may be, for example, a computer, a mobile device, a storage device, or the like. The device or system may include, for example, a file server for transmitting the computer program to the receiver.

In some embodiments, a programmable logic device (e.g., a field programmable gate array, FPGA) may be used to perform some or all of the functionalities of the methods described herein. In some embodiments, a field programmable gate array may cooperate with a microprocessor to perform any of the procedures described herein. In general, the methods are preferably performed by any hardware device.

Embodiments may be based on using an artificial intelligence, particularly a machine learning model or machine learning algorithm. Machine learning may refer to algorithms and statistical models that computer systems can use to perform a particular task without using explicit instructions, rather than relying on models and inference. For example, machine learning may use, instead of a rule-based transformation of data, a transformation of data that can be inferred from an analysis of history and/or training data. For example, the content of images may be analyzed using a machine learning model or using a machine learning algorithm. In order for the machine learning model to analyze the content of an image, the machine learning model may be trained using training images as input and training content information as output. By training the machine learning model with a large number of training images and/or training sequences (e.g., words or sentences) and associated training content information (e.g., labels or annotations), the machine learning model "learns" to recognize the content of the images so that the content of images not included in the training data can be recognized using the machine learning model. The same principle can be used for other types of sensor data as well: By training a machine learning model using training sensor data and a desired output, the machine learning model "learns" a transformation between the sensor data and the output, which can be used to provide an output based on non-training sensor data provided to the machine learning model. The provided data (e.g., sensor data, metadata, and/or image data) may be preprocessed to obtain a feature vector that is used as input to the machine learning model.

Machine learning models can be trained using training input data. The above examples use a training method called supervised learning. In supervised learning, the machine learning model is trained using a plurality of training samples, where each sample may include a plurality of input data values and a plurality of desired output values, i.e., each training sample is associated with a desired output value. By specifying both training samples and desired output values, the machine learning model "learns" which output value to provide based on an input sample that is similar to the samples provided during training. In addition to supervised learning, semi-supervised learning can also be used. In Semi-Supervised Learning, some of the training samples lack a desired output value. Supervised learning can be based on a supervised learning algorithm (e.g., a classification algorithm, a regression algorithm, or a similarity learning algorithm). Classification algorithms can be used when the outputs are restricted to a limited set of values (categorical variables), i.e., the input is classified as one of the limited set of values. Regression algorithms can be used when the outputs exhibit some numerical value (within a range). Similarity learning algorithms can be similar to both classification and regression algorithms, but are based on learning from examples using a similarity function that measures how similar or related two objects are. In addition to supervised learning or semi-supervised learning, unsupervised learning can be used to train the machine learning model. In Unsupervised Learning, (only) input data may be provided and an Unsupervised Learning algorithm may be used to find structure in the input data (e.g., by grouping or clustering the input data, finding commonalities in the data). Clustering is the assignment of input data comprising a plurality of input values into subsets (clusters) such that input values within the same cluster are similar according to one or more (predefined) similarity criteria, while they are dissimilar to input values comprised in other clusters.

Reinforcement learning is a third group of machine learning algorithms. In other words, reinforcement learning can be used to train the machine learning model. In reinforcement learning, one or more software actors (called "software agents") are trained to take actions in an environment. Based on the actions taken, a reward is calculated. Reinforcement learning is based on training the one or more software agents to select actions such that the cumulative reward is increased, resulting in software agents that become better at the task they are given (as evidenced by increasing rewards).

Further, some techniques can be applied to some of the machine learning algorithms. For example, feature learning can be used. In other words, the machine learning model may be trained at least in part using feature learning, and/or the machine learning algorithm may include a feature learning component. Feature learning algorithms, called representation learning algorithms, may preserve the information in their input but transform it in such a way that it becomes useful, often as a pre-processing stage before performing classification or prediction. Feature learning can be based on principal component analysis or cluster analysis, for example.

In some examples, anomaly detection (i.e., outlier detection) may be used, which aims to provide identification of input values that raise suspicion because they differ significantly from the majority of input and training data. In other words, the machine learning model may be trained at least in part using anomaly detection, and/or the machine learning algorithm may include an anomaly detection component.

In some examples, the machine learning algorithm may use a decision tree as a predictive model. In other words, the machine learning model may be based on a decision tree. In a decision tree, observations about an item (e.g., a set of input values) can be represented by the branches of the decision tree, and an output value corresponding to the item can be represented by the leaves of the decision tree. Decision trees can support both discrete values and continuous values as output values. When discrete values are used, the decision tree can be called a classification tree; when continuous values are used, the decision tree can be called a regression tree.

Association rules are another technique that can be used in machine learning algorithms. In other words, the machine learning model can be based on one or more association rules. Association rules are created by identifying relationships between variables given large amounts of data. The machine learning algorithm may identify and/or use one or more ratio rules that represent knowledge that is inferred from the data. The rules may be used, for example, to store, manipulate, or apply the knowledge.

Machine learning algorithms are typically based on a machine learning model. In other words, the term "machine learning algorithm" may refer to a set of instructions that can be used to create, train, or use a machine learning model. The term "machine learning model" may refer to a data structure and/or set of rules representing the learned knowledge (e.g., based on training performed by the machine learning algorithm). In embodiments, the use of a machine learning algorithm may imply the use of an underlying machine learning model (or a plurality of underlying machine learning models). The use of a machine learning model may imply that the machine learning model and/or the data structure/set of rules that is/are the machine learning model is trained by a machine learning algorithm.

For example, the machine learning model may be an artificial neural network (ANN; artificial neural network). ANNs are systems inspired by biological neural networks, such as those found in a retina or brain. ANNs include a plurality of interconnected nodes and a plurality of connections, called edges, between nodes. There are typically three types of nodes, input nodes that receive input values, hidden nodes that are connected (only) to other nodes, and output nodes that provide output values. Each node can represent an artificial neuron. Each edge can send information, from one node to another. The output of a node can be defined as a (nonlinear) function of its inputs (e.g., the sum of its inputs). The inputs of a node may be used in the function based on a "weight" of the edge or node providing the input. The weight of nodes and/or of edges can be adjusted in the learning process. In other words, training an artificial neural network may include adjusting the weights of the nodes and/or edges of the artificial neural network, i.e., to achieve a desired output for a given input.

Alternatively, the machine learning model may be a support vector machine, a random forest model, or a gradient boosting model. Support Vector Machines (i.e., support vector networks) are supervised learning models with associated learning algorithms that can be used to analyze data (e.g., in a classification or regression analysis). Support Vector Machines can be trained by providing an input with a plurality of training input values belonging to one of two categories. The Support Vector Machine can be trained to assign a new input value to one of the two categories. Alternatively, the machine learning model may be a Bayesian network, which is a probabilistic directed acyclic graphical model. A Bayesian network may represent a set of random variables and their conditional dependencies using a directed acyclic graph. Alternatively, the machine learning model can be based on a genetic algorithm, which is a search algorithm and heuristic technique that mimics the process of natural selection.

## Claims

1. A computer-implemented method for predictive periodontitis assessment, comprising the following steps:
predicting, using a first machine-learning model (1), a health status of a user regarding formation of gingivitis based at least in part on measurement data captured by a personal oral health care device used by the user; and
predicting, using a second machine-learning-model (2), a health status of the user regarding formation of periodontitis based at least in part on the predicted health status of the user regarding formation of gingivitis.

2. The method of claim 1, wherein the measurement data comprises personal brushing data relating to at least one brushing session performed by the user, the personal brushing data comprising one or more of: brushing frequency, brushing duration, brushing angle, brushing pressure, scrubbing behavior, and coverage of tooth segments.

3. The method of claim 1 or 2, wherein the predicting using the first machine-learning model is further based at least in part on plaque measurement data captured by the personal oral health care device used by the user.

4. The method of any one of the preceding claims, wherein the predicting using the first machine-learning model is further based at least in part on gingivitis measurement data captured by the personal oral health care device used by the user.

5. The method of any one of the preceding claims, wherein the predicting using the first machine-learning model is further based at least in part on information about the user, in particular demographics or comorbidities.

6. The method of any one of the preceding claims, further comprising:
causing output of an alarm or a recommendation for the user if the predicted health status of the user regarding formation of periodontitis exceeds a threshold;
wherein the alarm or recommendation is output by the personal oral health care device or an electronic user device associated therewith.

7. The method of any of the preceding claims, further comprising:
causing output of a control signal for changing and/or adapting at least one technical parameter of the personal health care device, particularly one or more comprising:
speed, such as brush motion speed;
pressure, such as brushing pressure and/or pressure exerted by a tooth brush head;
head orientation, in particular orientation of a tooth brush head with respect to the gum line;
intensity of a brushing and/or duration of a brushing time or session;
particularly for a selected part of the mouth and/or the gum line.

8. The method of any of the preceding claims, wherein the second machine-learning model models long-term aspects of periodontitis development using a supervised learning model and/or wherein using the second machine-learning model is not directly based on the measurement data captured by the personal oral health care device.

9. The method of any of the preceding claims, wherein the first machine-learning model and the second machine-learning model are and/or have been trained and/or validated independently of each other;
and/or wherein a training of the first machine-learning model is performed in real-time during operation of the personal oral health care device;
and/or wherein the measurement data is received from the personal oral health care device and/or fed into the first machine-learning model in real-time for purposes of the predicting the health status of a user regarding formation of gingivitis and/or for purposes of a training of the first machine-learning model.

10. The method of claim 7, wherein a feedback loop and/or regulatory circuit is arranged by:
feeding of the measurement data into the first machine-learning model;
the successive predicting using the first and/or second machine-learning model of a health status of a user regarding formation of gingivitis and/or periodontitis based at least in part on measurement data captured by a personal oral health care device used by the user; and
the causing of an output of a control signal for changing and/or adapting at least one technical parameter of the personal oral health care device.

11. A personal oral health care device, particularly toothbrush, a data processing device for a personal oral health care device, a controller for a data processing device for a personal oral health care device or a computer, comprising means for carrying out the method of any one of the preceding claims.

12. A data structure comprising health status data of a user regarding formation of periodontitis, obtained using the method of any one of the claims 1 - 10.

13. Use of the method of any one of the claims 1 - 10, the personal oral health care device of claim 11 and/or the data structure of claim 12 by an insurance and/or health care provider.

14. A computer program, or a computer-readable medium storing a computer program, the computer program comprising instructions which, when the program is executed on a computer and/or a computer network, cause the computer and/or the computer network to carry out the method of any one of claims 1-10.

15. A machine learning model structure for predictive periodontitis assessment, comprising a first machine-learning model (1) and a second machine-learning model (2),
wherein the first machine-learning model (1) is adapted to predict a health status of a user regarding formation of gingivitis based at least in part on measurement data captured by a personal oral health care device used by the user; and
wherein the second machine-learning-model (2) is adapted to predict a health status of the user regarding formation of periodontitis based at least in part on a predicted health status of the user regarding formation of gingivitis obtained using the first machine-learning model (1).
